# EUROPEAN PATENT APPLICATION

(11) **EP 4 692 035 A1**
(43) Date of publication of application: **11.02.2026**
(21) Application number: 24862986.7
(22) Date of filing: 04.07.2024
(51) Int. Cl.: C07C 29/76, C07C 29/80, C07C 29/04, C07C 31/10

(54) **METHOD OF PREPARING ISOPROPYL ALCOHOL**

(30) Priority: 08.09.2023 KR 20230119564; 28.06.2024 KR 20240085390
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR)
(74) Representative: Goddar, Heinz J.
(86) International application number: PCT/KR2024/009451
(87) International publication number: WO 2025/053403

(57) **Abstract**

The present invention provides a method of preparing isopropyl alcohol (IPA), and the method includes (S1) reacting propylene and water in a reaction unit to obtain a reaction product containing isopropyl alcohol (IPA); (S2) feeding the reaction product to a gas purification unit and separating gas components including unreacted propylene; and (S3) feeding the reaction product from which the gas components are separated to an IPA purification unit and obtaining purified isopropyl alcohol, wherein raw material propylene may be additionally fed to the gas purification unit, and the raw material propylene and the unreacted propylene may be purified.

## Description

### [Technical Field]

### CROSS-REFERENCE TO RELATED APPLICATION

The present application claims the benefit of priorities to Korean Patent Application No. 10-2023-0119564, filed on September 8, 2023 and Korean Patent Application No. 10-2024-0085390, filed on June 28, 2024, the entire contents of which are incorporated herein by reference.

### Technical Field

The present invention relates to a method of preparing isopropyl alcohol, and more particularly, to a method of preparing high purity isopropyl alcohol by purifying propylene used as a raw material together with unreacted propylene.

### [Background Art]

Isopropyl alcohol (IPA) is used for various purposes including a solvent for a cleaning agent, a raw material for an industrial paint or reagent, a paint, an ink, or the like in the electronics industry such as the manufacture of a semiconductor, a liquid crystal display (LCD), or the like.

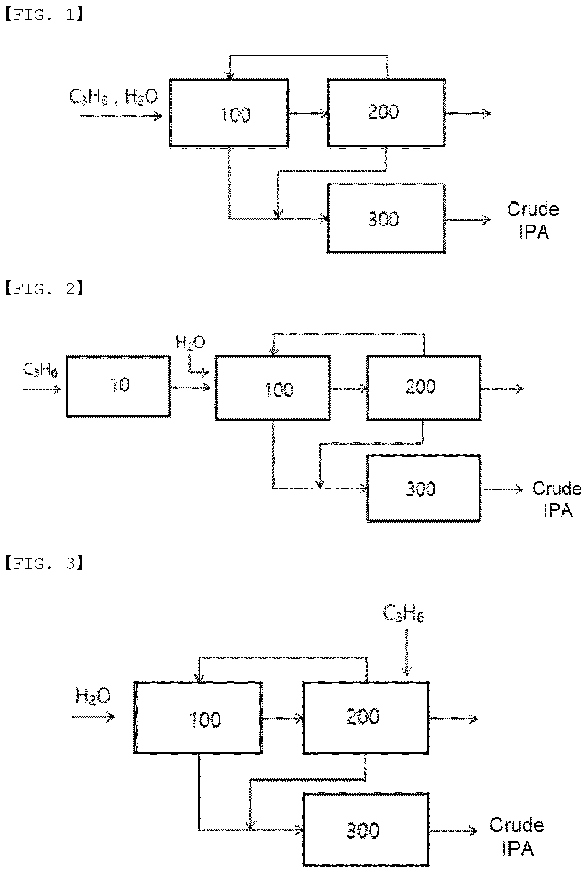

Such an isopropyl alcohol may be prepared by reacting propylene (C₃H₆) with water. Typically, as for isopropyl alcohol, referring to FIG. 1, a propylene monomer is reacted with water in a reaction unit 100 to obtain a reaction product containing an unreacted propylene monomer, unreacted water, and by-products such as n-propyl alcohol (NPA) and organic materials, along with IPA, the reaction product is transferred to a gas purification unit 200 to separate a gas containing low boiling point components including the unreacted propylene monomer, and then, the reaction product from which the gas components are separated is fed to an IPA purification unit 300 including a plurality of distillation columns to remove the organic materials, NPA, and water, thereby obtaining a crude isopropyl alcohol product.

It is preferable that propylene (C₃H₆) fed to the reaction unit as a raw material has high purity. In a case where impurities such as unsaturated hydrocarbons, for example, ethylene, butane, pentene, and the like, are contained in propylene, by-products (for example, ethanol) having a boiling point similar to that of IPA may be produced during the reaction between propylene and water. In addition, impurities having a boiling point similar to that of IPA, such as ethanol and n-propyl alcohol (NPA), may be already present in raw material propylene.

Therefore, purification of the raw material propylene is required to obtain high purity IPA. As illustrated in FIG. 2, in the existing IPA preparation process, a raw material purification unit 10 including two columns was installed upstream of the reaction unit to remove impurities contained in the raw material propylene.

However, when an IPA preparation system equipped with the raw material purification unit is operated to control the quality of the raw material propylene, energy consumption increases due to the use of steam, and facility costs and operating costs also increase due to the addition of columns.

### [Disclosure]

### [Technical Problem]

In order to solve the problems mentioned in the background art, the present invention relates to a method of preparing high purity isopropyl alcohol by purifying propylene used as a raw material together with unreacted propylene.

### [Technical Solution]

In one general aspect, a method of preparing isopropyl alcohol includes:
(S1) reacting propylene with water in a reaction unit to obtain a reaction product containing isopropyl alcohol (IPA);
(S2) feeding the reaction product to a gas purification unit and separating gas components including unreacted propylene; and
(S3) feeding the reaction product from which the gas components are separated to an IPA purification unit and obtaining purified isopropyl alcohol,
wherein raw material propylene is additionally fed to the gas purification unit, and the raw material propylene and the unreacted propylene are purified.

In the present invention, the raw material propylene may be fed at a higher position than the unreacted propylene in the gas purification tower.

### [Advantageous Effects]

According to the present invention, since the propylene used as a raw material and the unreacted propylene are simultaneously purified in the gas purification step of the isopropyl preparation process, there is no need to add a separate column for purifying the raw material propylene.

In addition, as the raw material propylene is fed at a higher position than the unreacted propylene in the gas purification tower, the purification of unreacted propylene having a high content of impurities such as propane and the purification of raw material propylene may be efficiently performed at the same time while minimizing energy consumption.

As described above, as the purification of raw material propylene and the purification of unreacted propylene are integrated, it is possible to not only reduce facility costs and operating costs associated with adding a separate column compared to the existing isopropyl preparation process, but also reduce energy consumption.

### [Description of Drawings]

FIG. 1 schematically illustrates a typical isopropyl alcohol preparation process.
FIG. 2 schematically illustrates a conventional isopropyl alcohol preparation process in which purification of raw material propylene is separately performed.
FIG. 3 schematically illustrates an isopropyl alcohol preparation process in which purification of raw material propylene and purification of unreacted propylene are integrated according to an embodiment of the present invention.
FIG. 4 illustrates the process of FIG. 3 in more detail.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

The meaning of "including" or "containing" used in the present application concretely specifies a specific property, region, integer, step, operation, element, and/or component, and does not exclude addition of another specific property, region, integer, step, operation, element, and/or component.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "upper portion" used in the present application refers to a point at a height of 0 to 20% from the upper end of a device to a lower portion, and specifically, may refer to the top (of the tower), unless otherwise specified. In addition, the term "lower portion" refers to a point at a height of 80 to 100% from the upper end of the device to the lower portion, and specifically, may refer to the bottom (of the tower).

The term "side stream" used in the present application may refer to a stream discharged at a height of 10 to 80% or 10 to 70% from the upper end of the device to the lower portion, unless otherwise specified.

In addition, the "pressure" referred to in the present application refers to gauge pressure measured based on atmospheric pressure.

Meanwhile, in the present invention, in devices such as an absorption tower, a purification tower, a removal tower, and a recovery tower, an operating temperature of the device may refer to a temperature of an upper portion or a lower portion of the device, unless otherwise specified. In addition, an operating pressure of the device may refer to a pressure of the upper portion of the device, unless otherwise specified.

An embodiment of the present invention relates to a method of preparing isopropyl alcohol (IPA). Hereinafter, the method of preparing IPA of the present invention will be described in detail with reference to the drawings.

FIG. 3 schematically illustrates an isopropyl alcohol preparation process in which purification of raw material propylene and purification of unreacted propylene are integrated according to an embodiment of the present invention.

Referring to FIG. 3, a method of preparing isopropyl alcohol according to the present invention includes a reaction step of propylene and water in a reaction unit 100 (S1); a purification step of unreacted propylene in a gas purification unit 200 (S2); and a step of obtaining high purity IPA in an IPA purification unit 300 (S3), wherein raw material propylene is fed to the gas purification unit and purified together with the unreacted propylene, and then fed to the reaction step.

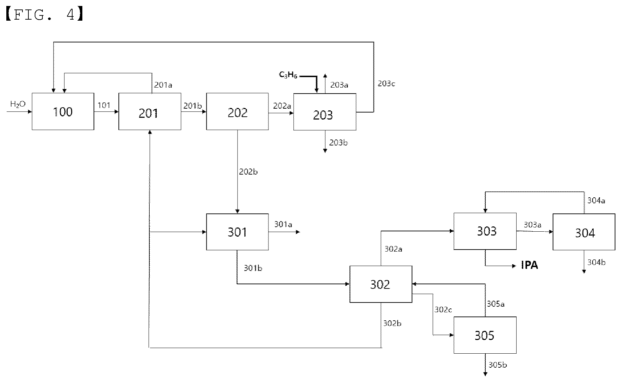

FIG. 4 illustrates an isopropyl alcohol preparation process according to an embodiment of the present invention in more detail. Isopropyl alcohol may be prepared using a system including a reaction unit 100; a gas purification unit including an absorption tower 201, a flash drum and gas separation tower 202, and a gas purification tower 203; and an IPA purification unit including an organic material removal tower 301, a water removal tower 302, an IPA recovery tower 303, a solvent recovery tower 304, and an NPA removal tower 305. In addition, the system may be used by additionally installing a reboiler for transferring heat to a feed stream of each tower and heating the feed stream, a condenser that converts an upper stream produced by the heating from a vapor phase to a liquid phase, a decanter for liquid-liquid separation of the condensed stream, a valve that controls a flow of a stream, a pump, and the like.

First, in the reaction unit 100, a reaction product 101 containing isopropyl alcohol may be obtained through a vapor phase reaction of a propylene monomer and water.

It is preferable that propylene (C₃H₆) fed to the reaction unit 100 as a raw material has high purity. In a case where impurities such as other unsaturated hydrocarbons, for example, ethylene, butane, pentene, and the like, ethane, propane, and carbon dioxide are contained in propylene, by-products (for example, ethanol) having a boiling point similar to that of IPA may be produced during the reaction between propylene and water.

Therefore, in order to obtain high purity IPA, purification of the raw material propylene is required, and in the present invention, the raw material propylene is purified in the gas purification unit positioned downstream of the reaction unit 100, specifically, in the gas purification tower 203, to remove impurities, and then fed to the reaction unit 100.

In an embodiment of the present invention, the purified propylene fed to the reaction unit 100 preferably contains, based on the total weight thereof, 97 wt% or more, for example, 97 to 99.8 wt% of propylene, and less than 3 wt% of impurities.

Meanwhile, only a part of the purified propylene fed to the reaction unit 100 is used in the reaction. Accordingly, the reaction product may contain an unreacted propylene monomer and unreacted water in addition to isopropyl alcohol produced by the reaction of the propylene monomer and water. For example, the reaction product 101 may contain 65 to 85 wt% of an unreacted propylene monomer, 4 to 8 wt% of isopropyl alcohol, and 5 to 30 wt% of water. In addition, the reaction product may contain isopropyl ether (DIPE), hexene, acetone, n-propyl alcohol (NPA), and the like as by-products. Therefore, there is a need to perform a process of separating unreacted raw materials from the reaction product and purifying isopropyl alcohol from various by-products.

To this end, the reaction product 101 obtained by the vapor phase reaction of the purified propylene and water is fed to the gas purification unit including the absorption tower 201, the flash drum and gas separation tower 202, and the gas purification tower 203, and gas components including an unreacted propylene monomer are separated (S2).

Specifically, the reaction product 101 is fed through a lower portion of the absorption tower 201, process circulating water is introduced through an upper portion of the absorption tower 201, and the unreacted propylene monomer is separated. In this case, as the process circulating water, water recovered in the distillation tower included in the downstream IPA purification unit, that is, the water removal tower 302, may be used. In the absorption tower 201, vapor phase isopropyl alcohol contained in the reaction product may be absorbed into the process circulating water and then obtained through a lower liquid phase stream 201b, and a vapor phase stream 201a containing an unreacted propylene monomer may be separated through the upper portion. The unreacted propylene monomer contained in the vapor phase stream 201a may be recycled to the reaction unit 100.

In an embodiment of the present invention, a flow rate of the process circulating water fed to the absorption tower 201 may be 15 to 40 wt% or 15 to 35 wt% of a flow rate of the reaction product. When the process washing water is supplied at a flow rate within the above range, absorption capacity of isopropyl alcohol contained in the reaction product may be improved, and at the same time, the energy cost for recovery of the process circulating water in the downstream process may be prevented from increasing excessively.

The absorption tower 201 may be operated at a temperature of 90 to 100°C or 90 to 95°C and a pressure of 25 to 40 kg/cm²·g or 25 to 35 kg/cm²·g. When the above operating conditions are satisfied, the upper discharge stream containing an unreacted propylene monomer and the lower discharge stream containing isopropyl alcohol may be effectively separated.

Meanwhile, the lower liquid phase stream of the absorption tower 201 may contain a small amount of the gas containing low boiling point components including an unreacted propylene monomer, which are not separated, in addition to isopropyl alcohol and unreacted water. For example, a content of the gas containing low boiling point components including an unreacted propylene monomer contained in the lower liquid phase stream of the absorption tower 201 may be 5 wt% or less or 2 to 5 wt%.

Accordingly, the liquid phase stream 201b containing isopropyl alcohol separated from the absorption tower 201 is fed to one or more flash drums and gas separation towers 202. An internal pressure in the flash drum may be reduced to 0 to 5 kg/cm²·g, and specifically, 0 to 2 kg/cm²·g, and the residual unreacted propylene contained in the liquid phase stream may be recovered as a gas and fed to the gas separation tower. The gas separation tower may be operated at an internal pressure of 10 to 30 kg/cm²·g, and specifically, 10 to 25 kg/cm²·g. In such a process, a vapor phase upper stream 202a containing residual unreacted propylene and a liquid phase lower stream 202b containing high boiling point components such as isopropyl alcohol may be discharged from the flash drum and gas separation tower 202.

The upper stream 202a discharged from the flash drum and gas separation tower may still contain inert gases including ethane, propane, and the like, which are impurities, and high boiling point components, in addition to unreacted propylene. For example, the stream 202a may contain 90 to 98 wt% of unreacted propylene and the balance of impurities based on the total weight of the stream 202a.

In order to remove the impurities contained in the unreacted propylene, the stream 202a containing unreacted propylene discharged from the flash drum and gas separation tower is fed to the gas purification tower 203.

In the present invention, the raw material propylene containing impurities is fed to the gas purification tower 203 and purified together with unreacted propylene. The raw material propylene fed to the gas purification tower may contain 95 to 99.8 wt% of propylene and the balance of impurities based on the total weight of the raw material propylene.

The purity of the raw material propylene is higher than that of the unreacted propylene. Therefore, in terms of separation performance, it is advantageous for the gas purification tower 203 to have a feeding port for raw material propylene positioned at a higher position than a feeding port for unreacted propylene containing a large amount of impurities such as propane.

In an embodiment of the present invention, the feeding port for raw material propylene may be disposed at a stage corresponding to a height of 25% to 60% from the upper end of the gas purification tower 203, and the feeding port for the stream 202a containing unreacted propylene may be disposed at a stage corresponding to a height of 50% to 85% from the upper end of the gas purification tower 203. When the feeding positions are satisfied, purification of unreacted propylene containing a large amount of impurities such as propane and purification of raw material propylene may be efficiently performed at the same time while minimizing energy consumption.

In addition, the stream containing unreacted propylene and the raw material propylene may be fed to the gas purification tower 203 in a flow rate ratio of 0.2:1 to 2:1, and specifically, 0.4:1 to 1.5:1, and when the above flow rate ratio range is satisfied, the removal efficiency of impurities may be increased while minimizing energy consumption.

The gas purification tower 203 may be operated at a temperature of 40 to 100°C, and specifically, 40 to 80°C, and a pressure of 10 to 30 kg/cm²·g, and specifically, 15 to 30 kg/cm²·g. When the above operating conditions are satisfied, impurities may be effectively removed from the unreacted propylene and the raw material propylene while minimizing energy consumption.

The raw material propylene and unreacted propylene purified in the gas purification tower 203 under the above conditions may contain 97 to 99.8 wt% of propylene and less than 3 wt% of the remaining impurities based on the total weight of the raw material propylene and unreacted propylene, and may be recovered through a side stream 203c and then recycled to the reaction unit 100. In addition, a stream 203a containing light components (for example, ethane and ethylene) separated from the gas purification tower 203 may be exhausted, and a stream 203b containing heavy components (for example, propane and butylene) may be discharged through a lower portion of the gas purification tower 203.

Meanwhile, the liquid phase stream 202b containing isopropyl alcohol discharged through a lower portion of the flash drum and gas separation tower 202 of the gas purification unit may contain isopropyl alcohol, water, and organic materials such as n-propyl alcohol (NPA), isopropyl ether (DIPE), and hexanol as by-products.

Therefore, the liquid phase stream 202b containing isopropyl alcohol needs to be purified into high purity isopropyl alcohol by passing through the IPA purification unit including the organic material removal tower 301, the water removal tower 302, the IPA recovery tower 303, the solvent recovery tower 304, and the NPA removal tower 305.

Specifically, the reaction product from which the gas components are separated, that is, the lower stream 202b discharged from the flash drum and gas separation tower of the gas purification unit, may be fed to the organic material removal tower 301 and brought into contact with process circulating water, and an upper stream 301a containing organic materials and a lower stream 301b containing isopropyl alcohol may be discharged. In this case, water recovered in the distillation tower included in the downstream IPA purification unit, that is, the water removal tower 302, may be used as the process circulating water, and the process circulating water may selectively dissolve isopropyl alcohol using a difference in solubility, allowing the isopropyl alcohol to move to the lower portion and to be separated from the organic by-products.

In an embodiment of the present invention, a flow rate of the process circulating water fed to the organic material removal tower 301 may be 20 to 50 wt% or 25 to 45 wt% of the flow rate of the reaction product. When the process washing water is supplied at a flow rate within the above range, a dissolution rate of isopropyl alcohol contained in the reaction product may be improved, and at the same time, the energy cost for recovery of the process circulating water in the downstream process may be prevented from increasing excessively.

Subsequently, the lower discharge stream 301b of the organic material removal tower is fed to the water removal tower 302, and an upper stream 302a containing isopropyl alcohol, a lower stream 302b containing water, and a side stream containing n-propyl alcohol (NPA) are separated and discharged.

The upper stream 302a separated from the water removal tower 302 may contain an azeotrope of isopropyl alcohol and water, and for example, may contain 80 to 90 wt% of isopropyl alcohol and 10 to 20 wt% of water.

The water separated through the lower stream 302b of the water removal tower 302 may be recovered and used as process circulating water. As described above, the water separated from the water removal tower 302 may be used as process circulating water fed to the upstream absorption tower 201 and organic material removal tower 301. In addition, the water separated from the water removal tower 302 may be used for heat exchange for preheating of water fed to the reaction unit 100, if necessary, because it has a high temperature exceeding 100°C.

Thereafter, the upper discharge stream 302a of the water removal tower 302 may be fed to the IPA recovery tower 303, an organic solvent (for example, cyclohexane, benzene, or the like) may be added as an azeotropic agent, and an upper stream 303a containing water and an organic solvent and a lower stream containing IPA may be discharged. That is, in the IPA recovery tower 303, the azeotrope of isopropyl alcohol and water is broken due to the organic solvent, such that isopropyl alcohol purified with high purity may be obtained.

The stream 303a containing water and an organic solvent, which is separated through an upper portion of the IPA recovery tower, may be fed to the solvent recovery tower 304, and an upper stream 304a containing a solvent and a lower stream 304b containing water may be separated and discharged. The solvent stream 304 may be recycled to the IPA recovery tower 303.

Meanwhile, a side discharge stream 302c of the water removal tower 302 containing n-propyl alcohol (NPA) may be fed to the NPA removal tower 305, a stream 305b containing NPA may be discharged through a lower portion of the NPA removal tower 305, and an upper stream 305a may be recycled to the water removal tower 302.

The operating conditions of the distillation towers included in the IPA purification unit are not particularly limited and may be appropriately selected within a commonly applicable range.

According to the present invention, since the propylene used as a raw material and the unreacted propylene are simultaneously purified in the gas purification step of the isopropyl preparation process, there is no need to add a separate column for purifying the raw material propylene.

In addition, as the raw material propylene is fed at a higher position than the unreacted propylene in the gas purification tower, the purification of unreacted propylene having a high content of impurities such as propane and the purification of raw material propylene may be efficiently performed at the same time while minimizing energy consumption.

As described above, as the purification of raw material propylene and the purification of unreacted propylene are integrated, it is possible to not only reduce facility costs and operating costs associated with adding a separate column compared to the existing isopropyl preparation process, but also reduce energy consumption.

### [Mode for Invention]

Hereinafter, the present invention will be described in more detail with reference to examples. However, the following examples are provided for illustrating the present invention. It is apparent to those skilled in the art that various modifications and alterations may be made without departing from the scope and spirit of the present invention, and the scope of the present invention is not limited thereto.

In the following examples and comparative examples, the method according to the present invention was simulated using the commercial process simulation program Aspen Plus. As the constants required for the simulation, the values stored in the program, the values described in the literature, and the like were used.

### Example 1

As illustrated in FIGS. 3 and 4, isopropyl alcohol was prepared using a system including a reaction unit 100; a gas purification unit 200 including an absorption tower 201, a flash drum and gas separation tower 202, and a gas purification tower 203; and an IPA purification unit 300 including an organic material removal tower 301, a water removal tower 302, an IPA recovery tower 303, a solvent recovery tower 304, and an NPA removal tower 305.

### (Step 1)

Water and the propylene purified in the downstream gas purification tower 203 were fed to the reaction unit 100, and a reaction was allowed to proceed, thereby obtaining a reaction product 101 containing isopropyl alcohol (IPA) as a product.

### (Step 2)

The reaction product 101 was fed to the absorption tower 201 of the gas purification unit 200, the process circulating water recovered in the downstream water removal tower 302 was introduced through an upper portion of the absorption tower 201, a liquid phase stream 201b into which vapor phase IPA was absorbed was discharged through a lower portion of the absorption tower 201, and a vapor phase stream 201a containing an unreacted propylene monomer was separated through the upper portion of the absorption tower 201 and recycled to the reaction unit 100. The absorption tower 201 was operated under conditions of a pressure of 33 kg/cm²·g and an upper/lower portion temperature of 95°C. The IPA-absorbed liquid phase stream 201b was fed to the flash drum and gas separation tower 202, the internal pressure of the flash drum was reduced to 0.5 kg/cm²·g, the gas separation tower was operated at a pressure of 20 kg/cm²·g, and a vapor phase upper stream 202a containing residual unreacted propylene and a liquid phase lower stream 202b containing IPA were discharged.

Subsequently, the vapor phase stream 202a containing unreacted propylene was fed to the gas purification tower 203, raw material propylene having the composition as shown in Table 1 was additionally fed to the gas purification tower 203, and the unreacted propylene and the raw material propylene were simultaneously purified (operating conditions: the pressure of 27 kg/cm²·g, the upper portion temperature of 45°C, and the lower portion temperature of 65°C). At this time, the vapor phase stream 202a containing unreacted propylene was fed at a position of 85% from the upper end of the gas purification tower 203, and the raw material propylene was fed at a position of 25% from the upper end, which is higher than the position for the vapor phase stream 202a.

The raw material propylene and unreacted propylene purified in the gas purification tower 203 were recovered through a side stream 203c and recycled to the reaction unit 100.

### (Step 3)

The liquid phase stream 202b containing isopropyl alcohol discharged through the lower portion of the flash drum and gas separation tower 202 was fed to the first organic material removal tower 301 of the IPA purification unit 300 and was brought into contact with the process circulating water recovered in the downstream water removal tower 302, and an upper stream 301a containing organic materials and a lower stream 301b containing isopropyl alcohol were discharged.

The lower discharge stream 301b of the organic material removal tower was fed to the water removal tower 302, an upper stream 302a containing isopropyl alcohol, a lower stream 302b containing water, and a side stream containing n-propyl alcohol (NPA) were separated and discharged, the upper discharge stream 302a of the water removal tower 302 was fed to the IPA recovery tower 303, cyclohexane was added as an azeotropic agent, an upper stream 303a containing water and cyclohexane was discharged, and IPA was separated through a lower portion of the IPA recovery tower 303, thereby obtaining a final IPA product.

Meanwhile, a side discharge stream 302c of the water removal tower 302 containing n-propyl alcohol (NPA) was fed to the NPA removal tower 305, a stream 305b containing NPA was discharged through a lower portion of the NPA removal tower 305, and an upper stream 305a was recycled to the water removal tower 302.

**[Table 1]**

| Composition of raw material propylene | |
|---|---|
| Ethane | 0.05 wt% |
| Propane | 0.45 wt% |
| Propylene | 99.2 wt% |

| | |
|---|---|
| Etc. | Ethylene 10 ppm, butane 5 ppm, butylene 5 ppm, and the like |

### Example 2

The same process as that of Example 1 was performed, except that, in Step 2, the vapor phase stream 202a containing unreacted propylene was fed at a position of 65% from the upper end of the gas purification tower 203, and the raw material propylene was fed at a position of 45% from the upper end, which is higher than the position for the vapor phase stream 202a.

### Example 3

The same process as that of Example 1 was performed, except that, in Step 2, the vapor phase stream 202a containing unreacted propylene was fed at a position of 45% from the upper end of the gas purification tower 203, and the raw material propylene was fed at a position of 65% from the upper end, which is lower than the position for the vapor phase stream 202a.

### Example 4

The same process as that of Example 1 was performed, except that, in Step 2, the vapor phase stream 202a containing unreacted propylene was fed at a position of 25% from the upper end of the gas purification tower 203, and the raw material propylene was fed at a position of 85% from the upper end, which is lower than the position for the vapor phase stream 202a.

### Comparative Example 1

### (Step 1)

Water and raw material propylene having the composition as shown in Table 1 were fed to the reaction unit 100, and a reaction was allowed to proceed, thereby obtaining a reaction product 101 containing IPA as a product.

### (Step 2)

The reaction product 101 was fed to the absorption tower 201 of the gas purification unit 200, the process circulating water recovered in the downstream water removal tower 302 was introduced through an upper portion of the absorption tower 201, a liquid phase stream 201b into which vapor phase IPA was absorbed was discharged through a lower portion of the absorption tower 201, and a vapor phase stream 201a containing an unreacted propylene monomer was separated through the upper portion of the absorption tower 201 and recycled to the reaction unit 100. The absorption tower 201 was operated under conditions of a pressure of 33 kg/cm²·g and an upper/lower portion temperature of 95°C. The IPA-absorbed liquid phase stream 201b was fed to the flash drum and gas separation tower 202, the internal pressure of the flash drum was reduced to 0.5 kg/cm²·g, the gas separation tower was operated at a pressure of 20 kg/cm²·g, and a vapor phase upper stream 202a containing residual unreacted propylene and a liquid phase lower stream 202b containing IPA were discharged.

Subsequently, the vapor phase stream 202a containing unreacted propylene was fed to the gas purification tower 203, the unreacted propylene was purified (operating conditions: the pressure of 27 kg/cm²·g, the upper portion temperature of 45°C, and the lower portion temperature of 65°C), and then, a side stream 203c containing the purified unreacted propylene was recovered and recycled to the reaction unit 100.

### (Step 3)

The same process as that of Step 3 of Example 1 was performed.

### Comparative Example 2

### (Step 1)

A raw material purification unit 10 including two columns was installed upstream of the reaction unit 100, and raw material propylene having the composition as shown in Table 1 was fed and purified (first column operating conditions: the pressure of 22 kg/cm²·g, the upper portion temperature of 45°C, and the lower portion temperature of 52°C; second column operating conditions: the pressure of 24 kg/cm²·g, the upper portion temperature of 52°C, and the lower portion temperature of 62°C).

Subsequently, water and the propylene purified in the raw material purification unit 10 were fed to the reaction unit 100, and a reaction was allowed to proceed, thereby obtaining a reaction product 101 containing IPA as a product.

### (Step 2)

The reaction product 101 was fed to the absorption tower 201 of the gas purification unit 200, the process circulating water recovered in the downstream water removal tower 302 was introduced through an upper portion of the absorption tower 201, a liquid phase stream 201b into which vapor phase IPA was absorbed was discharged through a lower portion of the absorption tower 201, and a vapor phase stream 201a containing an unreacted propylene monomer was separated through the upper portion of the absorption tower 201 and recycled to the reaction unit 100. The absorption tower 201 was operated under conditions of a pressure of 33 kg/cm²·g and an upper/lower portion temperature of 95°C. The IPA-absorbed liquid phase stream 201b was fed to the flash drum and gas separation tower 202, the internal pressure of the flash drum was reduced to 0.5 kg/cm²·g, the gas separation tower was operated at a pressure of 20 kg/cm²·g, and a vapor phase upper stream 202a containing residual unreacted propylene and a liquid phase lower stream 202b containing IPA were discharged.

Subsequently, the vapor phase stream 202a containing unreacted propylene was fed to the gas purification tower 203, the unreacted propylene was purified (operating conditions: the pressure of 27 kg/cm²·g, the upper portion temperature of 45°C, and the lower portion temperature of 65°C), and then, a side stream 203c containing the purified unreacted propylene was recovered and recycled to the reaction unit 100.

### (Step 3)

The same process as that of Step 3 of Example 1 was performed.

Table 2 shows the comparison of the IPA preparation processes and results according to examples and comparative examples.

**[Table 2]**

| | | Comparative Example 1 | Comparative Example 2 | Example 1 | Example 2 | Example 3 | Example 4 |
|---|---|---|---|---|---|---|---|
| Purification of raw material propylene | | - | Raw material purification unit | Gas purification tower | Gas purification tower | Gas purification tower | Gas purification tower |
| Feeding positions of raw material and unreacted propylene in gas ourification tower¹⁾ | | - | - | 25%/85% | 45%/65% | 65%45% | 85%/25% |
| Total energy consumed for purification of raw material and unreacted propylene²⁾ | | 0.34 | 1 | 0.93 | 0.45 | 1.57 | 2.32 |
| Composition of propylene recovered in gas purification tower (wt%) | Ethane | 1.07 | 0.19 | 0.19 | 0.19 | 0.19 | 0.19 |
| | Propylene | 94.05 | 97.81 | 97.81 | 97.81 | 97.81 | 97.81 |
| | Propane | 4.88 | 2.00 | 2.00 | 2.00 | 2.00 | 2.00 |
| Concentration of impurities (ethanol, outanol, and the like) in IPA product | | 6.4ppm | 0.8 ppm | 0.8 ppm | 0.8 ppm | 0.8 ppm | 0.8 ppm |
| 1) The feeding position in the gas purification tower 203 was set based on the height from the upper end (0%) to the lowermost portion (100%). | | | | | | | |
| 2) Energy consumption was simulated based on the composition of the upper stream 203c discharged from the gas purification tower of Comparative Example 2, and then, the total energy consumed for ourification of raw material and unreacted propylene was calculated. | | | | | | | |

In Table 2, in Examples 1 to 4, in which the purification of raw material propylene and the purification of unreacted propylene were simultaneously performed in the gas purification tower in the IPA preparation process, it was confirmed that the concentration of impurities in the propylene recovered in the gas purification tower and the finally obtained IPA product was reduced.

In particular, in Examples 1 and 2, in which the raw material propylene was fed at a higher position than the unreacted propylene, that is, at a position close to the upper end, in the gas purification tower 203, the separation performance was advantageous, and therefore, energy consumption was drastically reduced. Meanwhile, in Examples 3 and 4, since the raw material propylene was fed at a lower position than the unreacted propylene, that is, the feeding positions of the raw material propylene and unreacted propylene in the gas purification tower were not appropriate, energy consumption was high.

In Comparative Example 1, as the raw material propylene was introduced into the reaction unit 100 without purification, energy consumption was low, but a concentration of impurities in the propylene recovered in the gas purification tower 203 and the finally obtained IPA product was high due to the impurities contained in the raw material propylene. Therefore, it is difficult to use the IPA product as a high purity IPA product.

In Comparative Example 2, as the raw material propylene was purified in the separate raw material purification unit 10 and then fed to the reaction unit 100, a concentration of the impurities in the propylene recovered in the gas purification tower and the finally obtained IPA product was lowered, but as the number of columns required for the raw material purification unit 10 and the gas purification tower 203 was increased to three, the installation costs and operating costs increased compared to the examples in which the raw material propylene and unreacted propylene were simultaneously purified in the gas purification tower 203. In addition, energy consumption was also disadvantageous compared to Examples 1 and 2 in which the feeding positions of the raw material propylene and unreacted propylene were optimized.

### [Detailed Description of Main Elements]

- 10:: Raw material purification unit
- 100:: Reaction unit
- 200:: Gas purification unit
- 201:: Absorption tower
- 202:: Flash drum and gas separation tower
- 203:: Gas purification tower
- 300:: IPA purification unit
- 301:: Organic material removal tower
- 302:: Water removal tower
- 303:: IPA recovery tower
- 304:: Solvent recovery tower
- 305:: NPA removal tower

## Claims

1. A method of preparing isopropyl alcohol, the method comprising:
(S1) reacting propylene and water in a reaction unit to obtain a reaction product containing isopropyl alcohol (IPA);
(S2) feeding the reaction product to a gas purification unit and separating gas components including unreacted propylene; and
(S3) feeding the reaction product from which the gas components are separated to an IPA purification unit and obtaining purified isopropyl alcohol,
wherein a raw material propylene is additionally fed to the gas purification unit, and the raw material propylene and the unreacted propylene are purified.

2. The method of claim 1, wherein the gas purification unit includes an absorption tower, a flash drum, a gas separation tower, and a gas purification tower,
the reaction product fed through a lower portion of the absorption tower is brought into contact with process circulating water fed through an upper portion of the absorption tower, and a vapor phase upper stream containing unreacted propylene and a liquid phase lower stream containing components absorbed into the process circulating water are discharged from the absorption tower,
the lower discharge stream of the absorption column is fed to the flash drum and the gas separation tower, and a vapor phase upper stream containing residual unreacted propylene and a liquid phase lower stream containing isopropyl alcohol are discharged from the flash drum and the gas separation tower,
the vapor phase upper stream containing unreacted propylene discharged from the flash drum and the gas separation tower, and the raw material propylene are fed to the gas purification tower, a side stream containing purified propylene and a lower stream containing high boiling point components are discharged, and a gas containing low boiling point components are exhausted through an upper portion of the gas purification tower, and
the purified propylene separated from the gas purification tower is fed to the reaction unit.

3. The method of claim 2, wherein the raw material propylene is fed at a higher position than that of the unreacted propylene in the gas purification tower.

4. The method of claim 2, wherein a feeding port for the raw material propylene is fed at a stage corresponding to a height of 25% to 60% from the upper end of the gas purification tower, and
the stream containing unreacted propylene is fed at a stage corresponding to a height of 50% to 85% from the upper end of the gas purification tower.

5. The method of claim 2, wherein the stream containing unreacted propylene and the raw material propylene are fed in a flow rate ratio of 0.2:1 to 2:1, and specifically, 0.4:1 to 1.5:1.

6. The method of claim 2, wherein the gas purification tower is operated at a temperature of 40 to 100°C and a pressure of 10 to 30 kg/cm²·g.

7. The method of claim 2, wherein the raw material propylene fed to the gas purification tower contains 95 to 99.8 wt% of propylene based on the total weight of the raw material propylene.

8. The method of claim 2, wherein the raw material propylene and the unreacted propylene purified in the gas purification tower contain 97 to 99.8 wt% of propylene based on the total weight of the raw material propylene and the unreacted propylene.

9. The method of claim 2, wherein the gas containing low boiling point components exhausted from the gas purification tower includes ethane, ethylene, and a mixture thereof.

10. The method of claim 1, wherein the IPA purification unit includes an organic material removal tower, a water removal tower, an IPA recovery tower, an n-propyl alcohol removal tower, and a solvent recovery tower,
a liquid phase stream discharged from a flash drum and a gas separation tower of the gas purification unit is fed to the organic material removal tower and is brought into contact with process circulating water, and an upper stream containing organic materials and a lower stream containing isopropyl alcohol are discharged from the organic material removal tower,
a lower discharge stream of the organic material removal tower is fed to the water removal tower, and an upper stream containing a mixture of isopropyl alcohol and water, a lower stream containing water, and a side stream containing n-propyl alcohol (NPA) are discharged from the water removal tower,
an upper discharge stream of the water removal tower and an organic solvent are fed to the IPA recovery tower, and an upper stream containing water and an organic solvent and a lower stream containing IPA are discharged from the IPA recovery tower,
an upper discharge stream of the IPA separation tower is fed to the solvent recovery tower, and an upper stream containing a solvent and a lower stream containing water are discharged from the solvent recovery tower, and
a side discharge stream of the water removal tower is fed to the NPA removal tower, and a stream containing NPA is discharged through a lower portion of the NPA removal tower.
